(19) Europäisches Patentamt
European Patent Office
Office européen des brevets

(11) EP 2 879 647 B1

(12) # EUROPEAN PATENT SPECIFICATION

(45) Date of publication and mention of the grant of the patent:
**27.01.2021 Bulletin 2021/04**

(21) Application number: **13765797.9**

(22) Date of filing: **02.08.2013**

(51) Int Cl.:
***A61K 8/00*** *(2006.01)* ***A61K 36/61*** *(2006.01)*
***A61K 36/73*** *(2006.01)* ***A61P 17/10*** *(2006.01)*

(86) International application number:
**PCT/IB2013/056343**

(87) International publication number:
**WO 2014/020575 (06.02.2014 Gazette 2014/06)**

(54) **SKIN CARE**

HAUTPFLEGEMITTEL

SOIN DE LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.08.2012 ZA 201203221**

(43) Date of publication of application:
**10.06.2015 Bulletin 2015/24**

(73) Proprietor: **University of Pretoria**
**0083 Pretoria (ZA)**

(72) Inventors:
- **SHARMA, Richa**
  **Jodhpur (Rajasthan) 342003 (IN)**
- **LALL, Namrita**
  **0081 Pretoria (ZA)**
- **HUSSEIN, Ahmed**
  **7530 Bellville (ZA)**
- **KISHORE, Navneet**
  **Hillcrest 0083 Pretoria (ZA)**
- **MOODLEY, Indhrasen**
  **Road Entrance, 3**
  **Glenwood**
  **Durban 4041 (ZA)**

(74) Representative: **Brookes IP**
**Windsor House**
**6-10 Mount Ephraim Road**
**Tunbridge Wells, Kent TN1 1EE (GB)**

(56) References cited:

- **NAIR J J ET AL: "Anti-inflammatory effects of(Rosaceae) and identification of the active constituents", SOUTH AFRICAN JOURNAL OF BOTANY - SUID-AFRIKAANS TYDSKRIFT VIRPLANTKUNDE, FOUNDATION FOR EDUCATION, SCIENCE AND TECHNOLOGY, PRETORIA, SA, vol. 80, 23 February 2012 (2012-02-23), pages 75-76, XP028479235, ISSN: 0254-6299, DOI: 10.1016/J.SAJB.2012.02.009 [retrieved on 2012-03-14]**
- **ADEYEMI O AREMU ET AL: "Antioxidant activity, acetylcholinesterase inhibition, iridoid content and mutagenic evaluation of", FOOD AND CHEMICAL TOXICOLOGY, PERGAMON, GB, vol. 49, no. 5, 1 February 2011 (2011-02-01), pages 1122-1128, XP028158957, ISSN: 0278-6915, DOI: 10.1016/J.FCT.2011.02.003 [retrieved on 2011-02-08]**
- **A.O. AREMU ET AL: "In vitro antimicrobial, anthelmintic and cyclooxygenase-inhibitory activities and phytochemical analysis of Leucosidea sericea", JOURNAL OF ETHNOPHARMACOLOGY, vol. 131, no. 1, 1 August 2010 (2010-08-01), pages 22-27, XP055086756, ISSN: 0378-8741, DOI: 10.1016/j.jep.2010.05.043**
- **BOSMAN A A ET AL: "Isolation of an anthelmintic compound from Leucosidea sericea", SOUTH AFRICAN JOURNAL OF BOTANY - SUID-AFRIKAANS TYDSKRIFT VIRPLANTKUNDE, FOUNDATION FOR EDUCATION, SCIENCE AND TECHNOLOGY, PRETORIA, SA, vol. 70, no. 4, 1 October 2004 (2004-10-01), pages 509-511, XP009174000, ISSN: 0254-6299**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• D. NAIDOO ET AL: "Does micropropagation influence the antimicrobial properties of Bowiea volubilis?", SOUTH AFRICAN JOURNAL OF BOTANY, vol. 86, 1 May 2013 (2013-05-01), pages 157-158, XP055086753, ISSN: 0254-6299, DOI: 10.1016/j.sajb.2013.02.074

## Description

THIS INVENTION relates to skin care. In particular, the invention relates to a plant extract having *Propionibacterium acnes* inhibitory activity and compositions for use in the treatment of acne vulgaris.

## BACKGROUND OF THE INVENTION

Acne is an inflammatory disease caused by gram-positive bacterium *Propionibacterium acnes* (*P.acnes*). It is the most common skin disease that affects areas covering the oil glands and hair follicles usually found on the face, chest, upper arm, back and trunk (Leydon, 1997). *P. acnes* is an obligate anaerobic organism that has capability to activate and ability to metabolize sebaceous triglycerides into fatty acids inside sebaceous gland, due to increased production of sebum that causes thickening of epidermis at the outlet of pilosebaceous unit ensuing obstruction to flow of sebum outwards and a comedo develops (Chomnawang et al., 2012; Coenye et al., 2012). Due to increased fatty acids content, the production of various reactive oxygen species (ROS) from the damaged follicular walls led to the release of various cytokines like IL 8 and TNF a as host immune response. All these events lead to inflammation and pathogenesis of the disease. The usual drugs used in the treatment of acne have various side effects. The topical antibiotics can lead to dryness, redness, irritation of skin and hypopigmentation whereas oral antibiotics can cause gastrointestinal disorders and increase the risk of venous thromboembolism (Arican et al., 2005; Shaw and Kennedy, 2007).

The plant *Leucosidea sericea* Eckl. & Zeyh. (Rosaceae) is a single species of the genus *Leucosidea,* found in Eastern Cape, Free State and KwaZulu-Natal provinces of South Africa (Van Wyk et al., 2008).

The present invention aims to address the need for a natural and organic product, which can prevent the colonization of *P. acnes* on the skin.

In this specification reference is made to the following documents:


Arican, O., Kurutas, E.B., Sasmaz S., 2005. Oxidative stress in patients with acne vulgaris. Mediators Inflamm 6, 380-384.

Chomnawang, M.T, Surassmo, S., Nukoolkarn, V.S., Gritsanapan, W., 2005. Antimicrobial effects of Thai medicinal plants against acne-inducing bacteria. J Ethnopharmacol 101, 330-333.

Coenye, T., Brackman, G., Rigole, P., Witte, E.D., Honraet, K., Rossel, B., Nelis, H.J., 2012. Eradication of Propionibacterium acnes biofilms by plant extracts and putative identification of icariin, resveratrol and salidroside as active compounds Phytomedicine 19, 409- 412.

D'Hellencourt, C.L., Diaw, L., Cornillet, P., Guenounou, M., 1996. Differential regulation of TNFa, IL-1β, IL-6, IL-8, TNFβ and IL-10 by pentoxifylline. Int J Immunopharmac 18, 739-748.

Du Toit, R., Volsteedt, Y., Apostolides, Z., 2001. Comparison of the antioxidant content of the fruits, vegetables and teas measured as Vitamin C equivalents. Toxicology 166, 63-69.

Itoh, D., Kawano, K., Nabeta, K., 2003. Biosynthesis of chloroplastidic and extrachloroplastidic terpenoids in liverwort cultured cells: 13C serine as a probe of terpene biosynthesis via mevalonate and non-mevalonate pathways. J. Nat. Prod. 66, 332-336.

Kuete, V., 2010. Potential of Cameroonian plants and derived-products against microbial infections. A review. Planta Med 76, 1479-1491.

Leydon, J.J., 1997. Therapy for Acne vulgaris. The New England J. Med, 1156-1162.

Mapunya, M.B., Hussein, A.A., Rodriguez, B., Lall, N., 2011. Tyrosinase activity of Greyia flanaganii (bolus) constituents. Phytomedicine 18, 1006-1012.

Pan, C.Y., Chen, J.Y., Lin, T.L., Lin, C.H., 2009. In vitro activities of three synthetic peptides derived from epinecidin-1 and an anti-lipopolysaccharide factor against Propionibacterium acnes, Candida albicans, and Trichomonas vaginalis. Peptides 30, 1058-1068.

Rios, M.Y., Gonzalez-Morales, A., Villarreal, L., 2001. Sterols, triterpenes and biflavonoids of Viburnum jucundum and cytotoxic activity of ursolic acid. Planta Med 67, 683-684.

Shaw, L., Kennedy, C., 2007. The treatment of acne. J Paediatr Child Health 17, 385-389.

Suliman, S., Van Vuuren S.F., Viljoen, 2010. Validating the in vitro antimicrobial activity of Artemisia afra in polyherbal combinations to treat respiratory infections. S. Afr. J. Bot. 76, 655-661.

Sung, J.H., Lee, J.O., Son, J.K., Park, N.S., Kim, M.R., Kim, J.G., Moon, D.C., 1999. Cytotoxic constituents from Solidago virga-aurea var. gigantea MIQ. Arch Pharm Res 22(6), 633-637.

Tanghetti, A., 2005. Acne vulgaris and Rosacea: Optimizing Therapy. Skin Disease Education Foundation's 29th Annual Hawaii Dermatology Seminar, on March 18-24, in Maui, Hawaii.

Tsai, T.H., Tsai, T.H., Wu, W.H., Tseng, J.P., Tsai, P.J., 2010. In vitro antimicrobial and anti-inflammatory effects of herbs against Propionibacterium acnes. Food Chem 119, 964-968.

Tsai, I.L., Cheng, M.J., Hung, H.W., Cheng, H.I., Chen, I.S., 2007. Chemical constituents from the leaves of Litsea acutivena. J. Chinese Chem. Soc. 54, 503-506.

Van Wyk, B., Van Wyk, P., Van Wyk, B.E., 2008. Photo Guide to Trees of Southern Africa. Briza Publications, Pretoria, South Africa.

Lounasmaa, M., Widen, C.J., Huhtikangas, A., 1973. Phloroglucinol derivatives of Hagenia abyssinica. Phytochem. 12, 2017-2025.

Woldemariam, T.Z., Fell, A.F., Linley, P.A., Bibby, M.C., Phillips, R.M., 1992. Evaluation of the anti-tumour action and acute toxicity of kosins from Hagenia abyssinica. J. of Pharmaceut. Biomed. Anal. 10, 555-560.

## SUMMARY OF THE INVENTION

According to a first aspect of the disclosure there is provided a method for preparing a plant extract having *Propionibacterium acnes* inhibitory activity and skin hydration capability, the method includes the steps of
exposing plant material obtained from a *Leucosidea sericea* plant to a solvent to render an extraction solution; and
removing the plant material from the extraction solution thereby recovering an extract having *Propionibacterium acnes* inhibitory activity.

The step of exposing plant material obtained from a *Leucosidea sericea* plant to a solvent may comprise collecting plant material of *Leucosidea sericea,* grinding the plant material into a powder and soaking the powder in the solvent.

The step of removing the plant material from the extraction solution may comprise filtering the extraction solution at least once.

The method may further include a subsequent step of removing remaining solvent from the extraction solution thereby recovering a crude extract. The removal of the remaining solvent may comprise evaporating the solvent. The evaporation may take place under reduced pressure by a rotavapor.

In one embodiment of the disclosure the solvent may be in the form of water. The water may be in the form of autoclaved deionised water.

In another embodiment of the disclosure the solvent may be in the form of an organic solvent. The organic solvent may be in the form of any one ore more of diluted or undiluted solutions of: ethanol, methanol, ethyl acetate and acetone, and mixtures thereof.

The extract may include or be enriched for any one or more compounds selected from the group having the following structures:

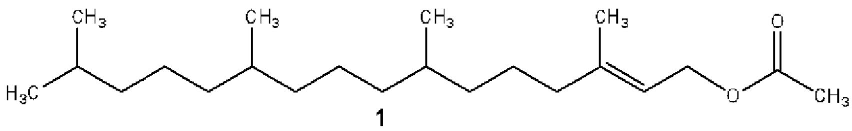

**1**

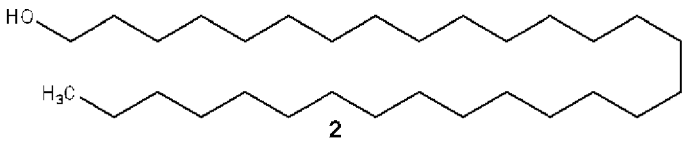

**2**

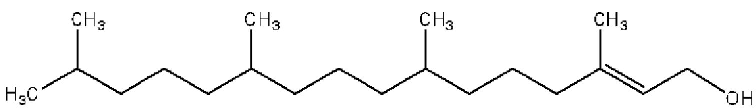

**3**

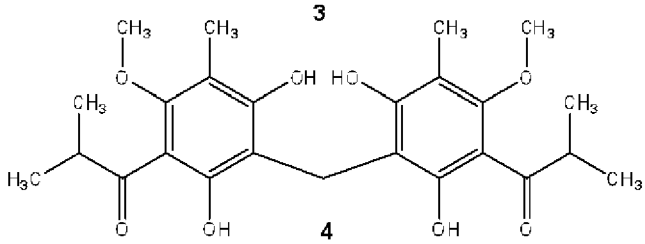

**4**

and derivatives thereof.

The extract may include, or be enriched for a *Propionibacterium acnes* inhibitory compound in the form of alpha kosin.

The plant material may be selected from any one or more of the group consisting of twigs and leaves of *Leucosidea sericea.* The leaves may be in the form of air-dried powdered leaves.

The plant material may be exposed to the solvent in a ratio of between 1:1 and 1:20 (w/v) plant material to solvent. The plant material may be exposed to the solvent in a ratio of between 1:2 and 1:10 (w/v) plant material to solvent. Preferably the plant material may be exposed to the solvent in a ratio of 1:4 (w/v) plant material to solvent.

The step of exposing the plant material to the solvent may comprise soaking the plant material in the solvent for at least two days. Preferably the plant material may be soaked in the solvent for at least three days.

The solvent may be removed from the extraction solution by drying the extraction solution under vacuum, following which the extract may be subjected to column fractioning using mixtures of hexane fraction (Hex): ethyl acetate (EtOAc) of increasing polarity (100:0 to 0:100) to obtain main fractions. The column fractioning may include 100 % methanol (MeOH) as eluent. The column may be in the form of a silica gel column.

The method of the disclosure may include the main fractions being subjected to further column fractioning. In one embodiment the further column fractioning may be in the form of silica gel column chromatography eluted with Hex: dichloromethane (DCM) mixtures of increasing polarity (100:0 to 0:100) to yield sub-fractions. In another embodiment the further column fractioning may be in the form of silica gel column chromatography eluted with mixtures of Hex: (DCM:MeOH::99:1) in equal ratio.

In accordance with another aspect of the disclosure, there is provided a method of preparing a plant extract enriched for a *Propionibacterium acnes* inhibitory compound in the form of alpha kosin, the method includes the steps of suspending plant material obtained from a *Leucosidea sericea* plant in a solvent, to render an extraction solution;
removing the plant material from the extraction solution; and
removing remaining solvent from the extraction solution, thereby recovering an extract enriched for a *Propionibacterium acnes* inhibitory compound.

The step of exposing plant material obtained from a *Leucosidea sericea* plant to a solvent may comprise collecting plant material of *Leucosidea sericea,* grinding the plant material into a powder and soaking the powder in the solvent.

The step of removing the plant material from the extraction solution may comprise filtering the extraction solution at least once.

The method may further include a subsequent step of removing remaining solvent from the extraction solution thereby recovering a crude extract. The removal of the remaining solvent may comprise evaporating the solvent. The evaporation may take place under reduced pressure by a rotavapor.

In one embodiment of the disclosure the solvent may be water. Particularly the water may be in the form of autoclaved deionised water. In another embodiment of the disclosure the solvent may be in the form of an organic solvent. The organic solvent may be any one or more solvents selected from the group consisting of diluted or undiluted solutions of: ethanol, methanol, ethyl acetate and acetone, and mixtures thereof.

The plant material may be selected from any one or more of the group consisting of twigs and leaves of *Leucosidea sericea.* The leaves may be in the form of air-dried powdered leaves.

The plant material may be exposed to the solvent in a ratio of between 1:1 and 1:20 (w/v) plant material to solvent. The plant material may be exposed to the solvent in a ratio of between 1:2 and 1:10 (w/v) plant material to solvent. In a preferred embodiment of the disclosure the plant material may be exposed to the solvent in a ratio of 1:4 (w/v) plant material to solvent.

The step of exposing the plant material to the solvent may comprise soaking the plant material in the solvent for at least two days. Particularly the plant material may be soaked in the solvent for at least three days.

The solvent may be removed from the extraction solution by drying the extraction solution under vacuum, following which the extract is subjected to column fractioning using mixtures of hexane fraction (Hex): ethyl acetate (EtOAc) of increasing polarity (100:0 to 0:100) to obtain main fractions. The column fractioning may include 100 % methanol (MeOH) as eluent. The column may be a silica gel column. The main fractions may be subjected to further column fractioning. The further column fractioning may be in the form of silica gel column chromatography eluted with mixtures of Hex: (DCM:MeOH::99:1) in equal ratio.

The invention also extends to an extract obtained using either of the methods described. The extract may increase skin hydration. The extract may inhibit the growth of *Propionibacterium acnes.* The extract may have a bacteriocidal effect on *Propionibacterium acnes.* The extract may exhibit anti-inflammatory activity by suppressing interleukin and tumour necrosis factor.

According to a first aspect of the invention, there is provided a composition comprising an extract of *Leucosidea Sericea,* in the form of either an ethanolic or liquid aqueous extract, for use in a method of treating acne vulgaris.

As a further aspect, there is provided a composition for use as detailed above, which further comprises a liquid aqueous extract of *Syzygium jambos.*

The invention further provides a composition comprising alpha kosin, for use in a method of treating acne vulgaris.

As a further aspect, the invention provides a topical composition for use in inhibiting the growth of *Propionibacterium acnes,* the topical composition comprising a liquid aqueous extract of *Leucosidea sericea* and *Syzygium jambos,* admixed with any one or more of an excipient, diluent, emollient and carrier. The topical composition may be admixed with any one of a pharmaceutical excipient, diluent and carrier to produce any one or more of an ointment, lotion, cream and emulsifying cream.

The invention will now be described, by way of example only with reference to the following figures:

## BRIEF DESCRIPTION OF THE FIGURES

In the Figures:

Figure 1 shows the structures of isolated compounds from *Leucosidea sericea.*

Figure 2 shows a transmission electron micrograph of a thin section of *P. acnes*: (a) untreated bacterias, labelled structures: cell wall (CW); cytoplasmic membrane (CM); nucleoid (N); ribosomes (R); mesosomes (M) x 60 K ; (b) *P. acnes* treated with *L. sericea* at 100 μg/ml x 60 K; (c) *P. acnes* treated with *L. sericea* at 300 μg/ml x 40 k; (d) *P. acnes* treated with compound C4 at 50 μg/ml x 50 K; (e) *P. acnes* treated with positive control (tetracycline) at 50 μg/ml x 60 K ; (f) *P. acnes* treated with solvent (DMSO at 2.5%) x 60 K. The arrows indicate cell injuries to the *P. acnes.*

Figure 3 shows a graph: (a) Dose-dependent inhibition of IL-8 and TNF a by ethanol extract of *Leucosidea sericea*; (b) Differential response of pentoxifylline on the release of IL-8 and TNF a by U937 cells infected with *P. acnes*

## DETAILED DESCRIPTION OF THE INVENTION

The invention relates to the potential of *Leucosidea sericea* Eckl. & Zeyh. addressing acne vulgaris. Four known compounds namely, phytol acetate, triacontanol, phytol and alpha kosin have been isolated for the first time from this plant. The ethanol extract of leaves and one of the isolated compounds, alpha kosin exhibited significant minimum inhibitory concentration (MIC) value of 15.7 and 1.9 μg/ml against acne inducing bacteria, *Propionibacterium acnes,* respectively. Moreover, the transmission electron micrographs showed the efflux of intracellular content to the cells of *P. acnes* caused by plant extract and alpha kosin. The ethanol extract of *L. sericea* exhibited significant anti-inflammatory activity by suppressing interleukin 8 (IL-8) and tumour necrosis factor (TNF a) in coculture of human U937 cells and heat killed *P. acnes.* The liquid aqueous extract of *L. sericea* inhibited the bacterial growth at 3% and demonstrated synergism and additive interaction when tested in combination with liquid aqueous extract of *Syzygium jambos* L. (Alston). The plant extract of *L. sericea* therefore has the potential as an alternative anti-acne agent.

**1.** Materials and Methods

1.1 General

Silica gel 60 (70-230 mesh); sephadex LH-20 and all the analytical grade chemicals were purchased from Sigma-Aldrich and Merck SA Pty Ltd. The ELIZA kit was purchased from BD Biosciences, Johannesburg, South Africa. Cell proliferation Kit II (XTT) was purchased from Roche diagnostics Pty Ltd., Johannesburg, South Africa. *Propionibacterium acnes* (ATCC 11827) was purchased from Anatech, Johannesburg, South Africa. The cell lines and medium were bought from Highveld Biological Pty Ltd., Johannesburg, South Africa.

### 1.2 Plant material

The twigs and leaves of *L. sericea* were collected from the botanical garden of University Of Pretoria, Pretoria, in March 2011. A voucher specimen (PRU 119052) was deposited at H.G.W.J Schwelckerdt Herbarium, Department of Plant Science, University of Pretoria, Pretoria.

### 1.3 Extraction and Isolation

The air-dried and powdered leaves (2.3 kg) were soaked in 9 L of ethanol for 3 days at room temperature. The filtrates were collected and concentrated under reduced pressure by a rotavapor at 40 °C to produce 73 g of crude ethanol extract. About 60 g of the ethanolic extract of *L. sericea* was subjected to silica gel column chromatography (70

cm× 120 cm) with hexane fraction (Hex): ethyl acetate (EtOAc) mixtures of increasing polarity (100:0 to 0:100) followed by 100% methanol (MeOH) as eluent. In total 51 fractions (500 ml) were collected and similar fractions were combined, according to thin-layer (TLC) profile, which resulted into 20 major fractions (MF). All the 20 major fractions were tested for antibacterial activity using broth dilution method against pathogenic *P. acnes.* The results are shown in Table 1. Fractions 6, 10, 15 and 16 showed inhibitory activity against *P. acnes*; hence were subjected further to chromatographic columns to isolate the bioactive compounds. MF 6 (600 mg) was separated on a silica gel column eluted with Hex: dichloromethane (DCM) mixtures of inventuiincreasing polarity (100:0 to 0:100) which yielded twenty three sub fractions (Sf). Sf 3-5 led to the isolation of **C1** (6 mg, 0.01%), Sf 7-8 eluted compound **C2** (10 mg, 0.02%) and Sf 9-12 led to the separation of compound **C3** (9 mg, 0.02%). The separation of MF 10 (1.4 g) was done using silica gel column chromatography eluting with mixture of Hex: (DCM: MeOH:: 99:1) in equal ratio, which yielded forty six Sf. Sf 4 and 5 were combined according to TLC analysis, consequently compound **C4** (34 mg, 0.06%) was obtained. Further, MF 15 and 16 (2.3 g) were combined based on TLC profile and were separated similar to MF 10, which yielded one hundred and forty Sf. From the Sf. 46-51, compound **C4** (15 mg, 0.03%) was obtained for the second time.

### 1.4 Antibacterial bioassay

The ethanol extract and compounds were tested against *P. acnes* by determining the minimum inhibitory concentration (MIC) values obtained by a microdilution method as previously described by Mapunya et al., (2011) with few modifications. Briefly, the bacteria was cultured from a Kwik-Stick on nutrient agar and incubated at 37°C for 72 h under anaerobic conditions before the assay. The 72 h culture of the bacteria was dissolved in nutrient broth and the suspension was adjusted to 0.5 McFarland standard turbidity. This resulted in $10^5$-$10^6$ colony forming units (CFU)/ml. In a sterile 96-well plate, 100 μl of samples from the stock solution consisting of the plant extract/ isolated compounds (2 mg/ml in 10% dimethyl sulphoxide (DMSO)) and the positive control tetracycline (0.2 mg/ml) was diluted with broth. Twofold serial dilutions were made in broth over a range to give concentrations of 500-3.9 μg/ml and 50-0.3 μg/ml for the plant extract/ isolated compounds and positive control tetracycline, respectively. The bacterial suspension (100 μl) was added to the wells. The wells with 2.5% DMSO and bacterial suspension without samples served as the solvent and negative controls respectively. The plates were incubated at 37°C for 72 h in an anaerobic environment. The MIC value was determined by observing colour change in the wells after addition of 2-(4-iodophenyl)-3-(4-nitrophenyl)-5-phenyl (INT) (defined as the lowest concentration that showed no bacterial growth).

### 1.5 Transmission electron microscopy (TEM)

The TEM procedures followed the protocol of a previous publication (Pan et al., 2009). Briefly, bacterial was concentrated by centrifugation at 10 000 rpm for 1 min. The pellet was resuspended in nutrient broth to a final OD550 nm of 1. The concentrations of plant extract were 1.3 and 4 times of its MIC; and 5 times of MIC for pure compound in order to visualise the lethal effects of tested samples against bacteria. The bacterial suspension (5 ml) was mixed with plant extract and pure compound to a final concentration of 300 and 100 μg/ml for plant extract and 50 μg/ml for pure compound. Tetracycline (50 μg/ml) and DMSO (2.5 %) were used as positive and solvent control. The pathogen was treated for 72 h; the control group consisted of only bacterial suspension in nutrient broth. Controls, treated and untreated *P. acnes* cultures were centrifuged and fixed in 2.5% glutraldehyde in phosphate buffer at room temperature for 1 h. Samples were washed with phosphate buffer and postfixed in both 1% osmium tetraoxide and uranyl acetate. The cells were dehydrated in ethanol and embedded in quetol resin. Thin sections were prepared with a microtome and micrographs were taken using a JEOL JEM-2100F field emission electron microscope.

### 1.6 Antioxidant assay

Antioxidant activity of the ethanol extract of *L. sericea* and purified compounds was investigated using 1, 2-diphenyl-1-picrylhydrazyl (DPPH) antioxidant assay. Following the procedures as described by DuToit et al. (2001) for each sample, a dilution series (8 dilutions) was prepared in a 96 well plate by adding distilled water as a dilution medium. Final concentration of the samples ranged from 100 to 0.7μg/ml. Each concentration was tested in triplicates. Vitamin C was used as a positive control. The radical scavenging capacities of the samples were determined using a BIOTEK plate reader to measure the disappearance of DPPH at 550 nm. The radical scavenging activity was measured in terms of the amount of antioxidants necessary to decrease the initial DPPH absorbance by 50% ($IC_{50}$). The $IC_{50}$ value of each sample was determined graphically by plotting the absorbance of DPPH as a function of the sample concentration in μg/ml. The $IC_{50}$ is the amount of antioxidant necessary to decrease the initial DPPH absorbance by 50%. The results were calculated using the following equation:

$$AA\% = \{Abs_{blank}(Abs_{sample} - Abs_{control}) / Abs_{blank}\} \times 100$$

The mg vitamin C equivalents/g dry weight of potent samples was calculated as follows:

$$VitEAC\ (mg\ AA/100\ g) = (IC_{50}(vit\ c)/IC_{50}(sample)) \times 1000.$$

**1.7 In vitro cytotoxicity assay**

The mouse melanocytes (B16-F10) cells were cultured in a complete Minimum Essential Eagle's Medium (MEM) whereas the human U937 cells were cultured in Roswell Park Memorial Institute (RPMI) containing 10% fetal bovine serum (FBS) and 1% gentamycin. B16-F10 ($10^5$ cells per well) and U937 ($10^6$ cells per well) were seeded into a 96-well plate. After an overnight incubation at 37 °C in 5% $CO_2$ and a humidified atmosphere, the extract, compounds and the positive control (actinomycin D) were added to the cells. The final concentrations of plant extract and pure compounds were ranging from 400-3.13 μg/ml and 100-1.5 μg/ml, respectively. The highest concentration of positive control (0.05 μg/ml) was serially diluted to eight consecutive wells. The plate was then incubated at 37°C in 5% $CO_2$, and a humidified atmosphere after which the toxicity effects of the extracts was assayed using the XTT (sodium 3'-[1-(phenyl amino-carbonyl)-3,4-tetrazolium]-bis-[4-methoxy-6-nitrobenzene sulfonic acid hydrate) cytotoxicity assay. Fifty micro litres of XTT reagent (1 mg/ml XTT with 0.383 mg/ml PMS) was added to the wells and incubated for 1 h. The optical densities of the wells were measured at 450 nm (690 nm reference wavelength) using BIOTEK Power-wave XS multi well reader (A.D.P., Weltevreden Park, South Africa). By referring to the control (medium with DMSO), the cell survival rate was assessed. The 'Graph Pad Prism 4', statistical program was used to analyze the 50% inhibitory concentration ($EC_{50}$) values.

**1.8 Preparation of heat-killed P. acnes and measurement of cytokine production**

The effect of plant extract on cytokine production (IL 8 and TNF a) was evaluated using enzyme immunoassay kits (ELIZA) using method as described previously (Tsai et al., 2010). Briefly, the log phase culture of *P.acnes* was harvested, washed three times with phosphate buffer saline (PBS), and incubated at 80°C for 30 min to kill the bacteria. The heat-killed bacteria were stored at 4°C until use. The human U937 cells were seeded at $10^6$ cells per well in a 24-well plate and was stimulated with heat killed *P. acnes* (wet weight 100 μg/ml) alone and in combination with the different test samples. Pentoxifylline was used as a control. After 18 h incubation, the cell-free supernatants were collected and the concentrations of IL 8 and TNF a were analysed. Cytokine standards were serially diluted to facilitate the construction of calibration curves necessary for determining protein concentration of test samples.

**1.9 Antibacterial activity of liquid aqueous extract and combination study**

**1.9.1 Preparation of liquid aqueous extract**

From our research, another plant namely, *Syzygium jambos* L. (Alston) (Myrtaceae) was found to be active against *P.acnes.* Therefore, it was decided to investigate the antibacterial activity of liquid aqueous extract of *S. jambos* in combination with *L. sericea.* The liquid aqueous extract of *L. sericea* and *S. jambos* was prepared by weighing 12.5 g of dried leaves powder and soaking in 150 ml of autoclaved deionised water. The soaked leaves were kept on a shaker for 3 days and then were filtered four times to obtain the liq aq. extract. Euxyl 9010 (1%) was added as preservative.

**1.9.2 Antibacterial activity of liquid aqueous extract**

The antibacterial activity of liq aq. extract of *L. sericea* and *S. jambos* was carried out as explained in section 2.4 with few modifications. Briefly, the bacteria was cultured from a Kwik-Stick on nutrient agar and incubated at 37°C for 72 h under anaerobic conditions before the assay. The 72 h culture of the bacteria was dissolved in nutrient broth and the suspension was adjusted to 0.5 McFarland standard turbidity. This resulted in $10^5$-$10^6$ colony forming units (CFU)/ml. In a sterile 96-well plate, 100 μl of sterile liq aq. extract (sterilised by micro filter with 0.2 micron pore size) was diluted with 100 μl of broth. The serial dilutions were made in the broth to give percentage concentrations ranging from 25% - 0.19%. Tetracycline was used as positive control and was serially diluted to give concentration ranging from 50 - 0.3 and μg/ml. The bacterial suspension (100 μl) was added to the wells. The bacterial suspension without samples served as the negative control. The plates were incubated at 37°C for 72 h in an anaerobic environment. The MIC value

was determined by observing colour change in the wells after the addition of INT (defined as the lowest concentration that showed no bacterial growth).

**1.9.3 Antibacterial activity in combination**

The antibacterial activity in combination was done following the same procedure as explained in section 1.9.2. The liquid aqueous extract of *Leucosidea sericea* (LS) and *Syzygium jambos* (SJ) were combined in different ratios to make nine combinations (LS:SJ :: 1:9, 2:8, 3:7, 4:6, 5:5, 6:4, 7:3, 8:2, 9:1). The activity of plants in combination was evaluated by calculating the Fraction Inhibitory Concentration (FIC) value (Suliman et al., 2010). The MIC value of liquid aqueous. extract of *Leucosidea sericea* (LS) and *Syzygium jambos* (SJ) alone and in nine different combinations were used to calculate FIC by the following equation:

$$\text{FIC 1} = \text{MIC (a) in combination with (b) / MIC (a) independently}$$

$$\text{FIC 2} = \text{MIC (a) in combination with (b) / MIC (b) independently}$$

Where (a) represents *L. sericea* and (b) represents *S. jambos.* The FIC of all the combinations was calculated and sum of FIC known as FIC index was calculated by adding all the FIC.

The FIC index was used to determine the correlation of the two plants in combination and may be classified as synergistic (≤ 0.5), additive (> 0.5-1.0), non-interactive (> 1.0- ≤ 4.0) or antagonistic (> 4.0).

**1.10 Clinical studies**

The liquid aqueous extract of *L. sericea* and a combination of *L. sericea* and *S. jambos* in ratio 1:1 were selected for the following clinical studies.

**1.10.1 Patch testing**

A 24 h occlusive irritancy patch testing was performed on 20 subjects.

**1.10.2 In Vivo Soothing and Calming efficacy**

An in vivo study to determine the soothing and calming efficacy of the samples for 24 to 72 hours post-application on tape stripped skin was done.

**2. Result and discussion**

**2.1 Identification of isolated compounds**

The ethanol extract of dried and powdered leaves of *L. sericea* subjected to chromatographic purification resulted into isolation of four known compounds **(C1-C4)** (Fig. 1). Structural assessment of these compounds was characterized by Mass, $^{1}$H and $^{13}$C NMR spectroscopic data. Assignment of signals was facilitated by COSY, HSQC and HMBC experiments. The four known compounds obtained in this study, phytol acetate **(C1)** (Itoh et al., 2003), triacontanol **(C2),** (Tsai et al., 2007), phytol **(C3)** (Itoh et al., 2003), and alpha kosin **(C4)** (Woldemariam et al., 1992) were identified by comparison of their physical and spectroscopic data with literature reports and are isolated for the first time from *L. sericea.*

**2.2 Antibacterial bioassay**

The antibacterial activity of ethanol extract of *L. sericea* and isolated compounds have been summarised in Table 1. The ethanol extract of *L. sericea* inhibited the bacterial growth and exhibited noteworthy MIC value of 15.6 μg/ml. Compound **C4** was found to be the most active compound against *P. acnes* with MIC value of 1.9 μg/ml as compared to tetracycline (positive control) with MIC value of 3.1 μg/ml. It is worth noting that threshold MIC values of 100 and 10 μg/ml have been recommended for plant extracts and pure compounds, respectively, to rate them as having significant antimicrobial activity (Kuete, 2010). Thus the MIC values measured for the activity of crude extract and

compound **C4** can be considered significant. Compounds 1-3 did not showed any growth inhibitory activities at highest concentration tested (500 μg/ml). To the best of our knowledge, the antibacterial activity of *L. sericea* and isolated compounds **(C1-C4)** against *P. acnes* is being reported for the first time.

**Table 1: Antibacterial, antioxidant and cytotoxic effects of ethanol extract of *Leucosidea sericea* and isolated compounds**

| **Test samples** | **Antibacterial** MIC μg/ml/(μ**M**) | **Antioxidant $IC_{50}$** μg/ml/(μ**M**) | **Cytotoxicity $EC_{50}$** μg/ml/(μ**M**) | |
|---|---|---|---|---|
| | | | B16-F10 mouse melanocytes | U937 human macrophage |
| *Leucosidea sericea* | 15.6 | 2.01 | 55.51 | 26.03 |
| MF 1-5,7-9, 11-14, 17-20 | [a]Na | [b]- | - | - |
| MF6 | 500 | - | - | - |
| MF 10 | 1.9 | - | - | - |
| MF 15, 16 | 31.3 | - | - | - |
| **C1** (phytol acetate) | Na | >100 | [c]Nt | Nt |
| **C2** (triacontanol) | Na | >100 | >100 | >100 |
| **C3** (Phytol) | Na | >100 | >100 | >100 |
| **C4** (alpha kosin) | 1.9/**(2.1)** | 5.05/**(10.5)** | < 3.12 | <3.12 |
| [d]**PC** | 3.1/(**7**) | 1.98/(**11.3**) | 4.5 x $10^{-3}$/**3.5 x $10^{6}$** | 4.5 x $10^{-3}$/**3.5 x $10^{6}$** |

[a]not active at the highest concentration tested (500 μg/ml)
[b]not applicable
[c]not tested due to low yield.
[d]positive drug control where tetracycline for antibacterial, vitamin C for antioxidant, actinomycin D for cytotoxicity

**2.3 Transmission electron microscopy (TEM)**

For microscopy studies, the plant extract and compound **C4** which showed activity against the bacteria were selected. The TEM micrograph represents clear differences between untreated and treated *P. acnes.* The untreated *P. acnes* showed normal morphological characters with distinct cell wall which was long, spindle shaped, smooth and lined with cell membrane. A centrally located nucleoid surrounded by ribosomes was observed (Fig 2a). The TEM micrograph showed cell injuries caused to *P. acnes* on exposure to the ethanol extract of *L. sericea* for 72 h. At a concentration of 100 μg/ml, *P. acnes* exhibited abnormal changes in cell content material (Fig 2b) whereas at higher concentration of 300 μg/ml, the cell wall of bacteria was found lysed and cell debris was observed (Fig 2c). Compound **C4** caused significant damage to *P. acnes* at 50 μg/ml. The intracellular content was found to be effluxed due to breaks in the cell wall. The intact cells showed changes in the appearance of cell organelles. Due to extensive lysis of the bacterias, the debris was observed all over (Fig 2d). Tetracycline at a concentration of 50 μg/ml, caused significant damages to the cells of *P. acnes,* leading to damages in the cell membrane, distortion in the cell structure and shrinkage of cell content material (Fig 2e). DMSO at 2.5% exhibited no lethal effects to bacteria (Fig 2f). The TEM micrograph confirms the antibacterial activity of *L. sericea* and **C4** against *P. acnes.*

**2.4 Antioxidant assay**

DPPH assay provides radical scavenging properties of samples. The antioxidants are able to stable the free DPPH radical due to their proton donating ability. The scavenging effect of *L. sericea* ethanol extract and compound **C5** on DPPH increased with their increasing concentrations. These samples showed very significant antioxidant activity

with $IC_{50}$ values very similar to Vitamin C, a widely used antioxidant compound. The results are shown in Table 1. The mg vitamin C equivalents/g dry weight for **C5** was calculated to be 392. Compounds **C1-C3** did not showed any antioxidant activity. Concerning the structure-activity relationship, it is clear that the presence of four free hydroxyl groups in **C4** are responsible for its antioxidant activity in comparison with **C1-C3** which possess less or lacks any free hydroxyl groups. Similar to our results, the methanol leaves extract of *L.sericea* exhibited antioxidant potential with $IC_{50}$ value of 3.0 μg/ml. However, petroleum ether and DCM extract exhibited a higher $EC_{50}$ value of 26.2 and 27.7 μg/ml, respectively (Aremu et al., 2010). To the best of our knowledge, this is the first report of DPPH scavenging activity of all the compounds isolated in this study.

### 2.5 *In vitro* cytotoxicity assay

The cytotoxicity of the extracts and compound **C2, C3** and **C4** was done on B16-F10 mouse melanocytes and U937 human macrophage cells. Due to low yield of **C1** no further test were done on them. All the results are shown in Table 1. To the best of our knowledge, the cytotoxicity in the present study is reported for the first time. *L. sericea* exhibited moderate toxicity to B16-F10 cells and comparatively higher toxicity to U937 cells. **C4** showed significant toxicity to both the cell lines with $EC_{50}$ value of below then 3.12 μg/ml. However, **C2** and **C3** did not exhibit any toxicity to both the cell lines with 100% viability of cells at highest concentration of 100 μg/ml tested. Similar to our findings, strong cytotoxic effects of alpha kosin against MAC tumour cells are reported by Woldemariam et al. (1992). Triacontanol was reported as non toxic constituent of Viburnum *jucundum* (Rios et al., 2001). Phytol showed mild toxicity against skin cancer cells (SL-MEL-2), CNS cancer cells (XF498) and colorectal cancer cells (HCT15) (Sung et al., 1999). No reports about cell toxicity have been found for *L.sericea* in the literature.

### 2.6 Anti-inflammatory activity

*P. acnes* stimulate macrophages for increased production of pro-inflammatory cytokines such as IL-8 and TNF a which contributes to the induction of mediators of inflammatory response. In the present study, IL-8 and TNF a were used as major criteria for evaluation of anti-inflammatory activity. To test the anti-inflammatory effects of *L.sericea* an *in vitro* screening at three non toxic concentrations were applied. The U937 cells co-cultured with *P. acnes* caused an increase in the production of IL-8 and TNF a (Fig 3a). As shown in Fig 3a, the ethanol extract of *L. sericea* decreased the production of IL-8 and TNF a in dose-dependent manner. Furthermore, the plant extract did not increase the secretion of either of the cytokines in culture of U937 cells in the absence of heat killed *P. acnes* (data not shown). Pentoxifylline which was used as a control behaves differently on the cytokines. Based on previous reports, it downregulates the secretion of TNF a and cause no change in IL-8 release (D'Hellencourt et al., 1996). As shown in Fig 3b, our results were in agreement with other researchers. Significant inhibition of TNF a was observed at 100 and 50 μg/ml of pentoxifylline whereas, no change in IL-8 concentration was observed. To the best of our knowledge, no reports about *L.sericea* in context with suppression of cytokines were found. Although, similar to our results, other plants such as *Eucommia ulmoides* and *Ilex paraguariensis* extracts were reported to reduce the secretion of IL 8 and TNF a in human monocytic THP-1 cells pre-treated with *P. acnes* at concentration of 0.1 mg/ml (Tsai et al., 2010).

### 2.7 Antibacterial activity of liquid aqueous extract

The MIC value of liquid aqueous extract of *Leucosidea sericea* and *Syzygium jambos* individually and in nine combinations is summarised in Table 2. All the combinations where *L. sericea* was dominant showed additive interactions with FIC index value of 0.72. The other combinations of LS: SJ where S. *jambos* was dominant and in equal ratio to *L. sericea* showed synergism with FIC index value of 0.33. The results of MIC and FIC index values of *L. sericea* and S. *jambos* shows that the liquid aqueous extract of both these plants have exhibited improved antibacterial activity when tested in combination.

**Table 2: MIC and FIC index values of liquid aqueous extract *Syzygium jambos* and *Leucosidea sericea***

| Plant samples | MIC (%) | Σ FIC |
|---|---|---|
| *L. sericea* | 3.1 | |
| *S. jambos* | 6.2 | |
| **Combinations LS:SJ ratios** | | |
| 9:1,8:2, 7:3, 6:4 | 1.5 | 0.72 |
| 5:5, 4:6, 3:7, 2:8, 1:9 | 0.7 | 0.33 |

### 2.8 Clinical studies

It was found that the liquid aqueous extract of *Leucosidea sericea* alone and in combination with *Syzygium jambos* was not an irritant. The *in vivo* soothing and calming efficacy was tested and the liquid aqueous extract of *Leucosidea sericea* alone and in combination with *Syzygium jambos* showed significant increase in hydration after 24 h at 5% level of confidence. To the best of our knowledge, this is the first report on hydration and skin irritancy for the selected samples.

Acne affects barrier function directly through the impact of the inflammatory process on epidermal growth and maturation. Medications used to treat a number of conditions indirectly disrupt barrier function. Conventional acne therapeutics such as benzoyl proxide and retinoic acid decreases stratum corneun hydration leading to a dry and itchy skin. Hydration therefore strongly benefits the acne prone skin. A skin hydrating agent acts as stabilized epidermal barrier which means less potential for skin irritation. Restoring and maintaining barrier function is critical in patients with dermatological diseases. The addition of moisturizers/hydration calming agents to acne treatment products helps maintain barrier function and will provide better outcomes. For comedonal acne, combination products containing moisturizers/hydrating agents may be effective first-line agents (Tanghetti, 2005). The liquid aqueous extract of *L. sericea* alone and in combination with *S. jambos* therefore could be an ideal concomitant alternative topical treatment for acne.

### 3. Conclusion

The ethanol extract of *L. sericea* and isolated compound alpha kosin demonstrated significant activity against *P. acnes* which was also confirmed by TEM micrographs. The plant extract exhibited noteworthy antioxidant and anti-inflammatory activity which are crucial for an anti-acne agent. The liquid aqueous extract of *L. sericea* and *S. jambos* also inhibited the bacterial growth but the combination study showed better results where synergism and additive interactions were observed. It was interesting to note that no antagonism was evident. The clinical studies showed significant improvement of hydration (24 h) by liquid aqueous extract of *L. sericea* and liquid aqueous extract of *L. sericea* and *S. jambos* in ratio 1:1. The plant therefore has potential as alternative anti-acne agent.

## Claims

**1.** A composition comprising an extract of *Leucosidea Sericea,* in the form of either an ethanolic or liquid aqueous extract, for use in a method of treating acne vulgaris.

**2.** The composition for use as claimed in claim 1, which further comprises a liquid aqueous extract of *Syzygium jambos.*

**3.** A composition comprising alpha kosin, for use in a method of treating acne vulgaris.

**4.** A topical composition for use in inhibiting the growth of *Propionibacterium acnes,* the topical composition comprising a liquid aqueous extract of *Leucosidea sericea* and *Syzygium jambos,* admixed with any one or more of an excipient, diluent, emollient and carrier.

**5.** The topical composition for use as claimed in claim 4, wherein the topical composition is admixed with any one of a pharmaceutical excipient, diluent and carrier, to produce any one or more of an ointment, lotion, cream and emulsifying cream.

## Patentansprüche

**1.** Zusammensetzung, die einen Extrakt von *Leucosidea sericea* in Form entweder eines ethanolischen oder eines flüssigen wässrigen Extrakts umfasst, zur Verwendung in einem Verfahren zur Behandlung von Acne vulgaris.

**2.** Zusammensetzung zur Verwendung gemäß Anspruch 1, die weiterhin einen flüssigen wässrigen Extrakt von *Syzygium jambos* umfasst.

**3.** Zusammensetzung, die alpha-Kosin umfasst, zur Verwendung in einem Verfahren zur Behandlung von Acne vulgaris.

**4.** Topische Zusammensetzung zur Verwendung bei der Hemmung des Wachstums von *Propionibacterium acnes,*

wobei die topische Zusammensetzung einen flüssigen wässrigen Extrakt von *Leucosidea sericea* und *Syzygium jambos* umfasst, der mit einem oder mehreren aus einem Arzneimittelhilfsstoff, Verdünnungsmittel, Weichmacher und Träger vermischt ist.

**5.** Topische Zusammensetzung zur Verwendung gemäß Anspruch 4, wobei die topische Zusammensetzung mit irgendeinem aus einem Arzneimittelhilfsstoff, Verdünnungsmittel und Träger vermischt ist, wobei eines oder mehrere aus einer Salbe, Lotion, Creme und emulgierenden Creme erzeugt werden.

## Revendications

**1.** Composition comprenant un extrait de *Leucosidea Sericea,* sous la forme d'un extrait soit éthanolique soit aqueux liquide, à utiliser dans un procédé de traitement de l'acné vulgaire.

**2.** Composition à utiliser selon la revendication 1, qui comprend en outre un extrait aqueux liquide de *Syzygium jambos.*

**3.** Composition comprenant de l'alpha-kosine, à utiliser dans un procédé de traitement de l'acné vulgaire.

**4.** Composition topique à utiliser dans l'inhibition de la croissance de *Propionibacterium acnes,* la composition topique comprenant un extrait aqueux liquide de *Leucosidea sericea* et de *Syzygium jambos,* mélangé avec un ou plusieurs parmi excipient, diluent, émollient et vecteur.

**5.** Composition topique à utiliser selon la revendication 4, dans laquelle la composition topique est mélangée avec l'un quelconque d'un excipient, diluent et vecteur pharmaceutique, pour produire une ou plusieurs parmi pommade, lotion, crème et crème émulsifiante.

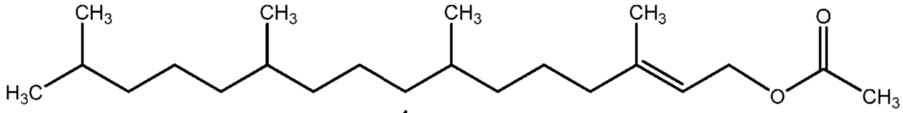

**1**

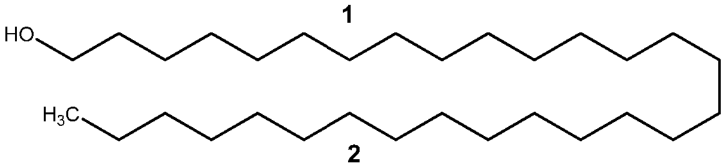

**2**

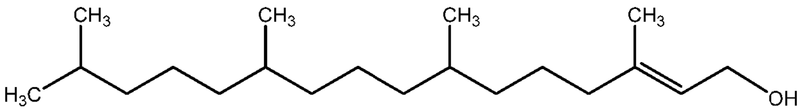

**3**

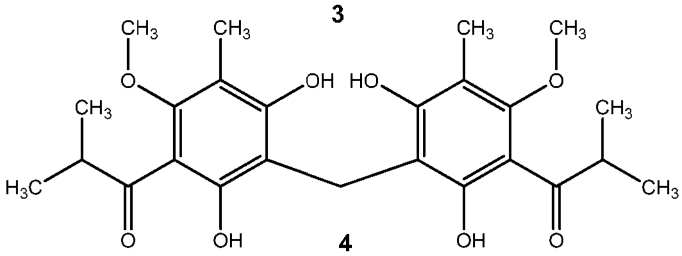

**4**

**Figure 1**

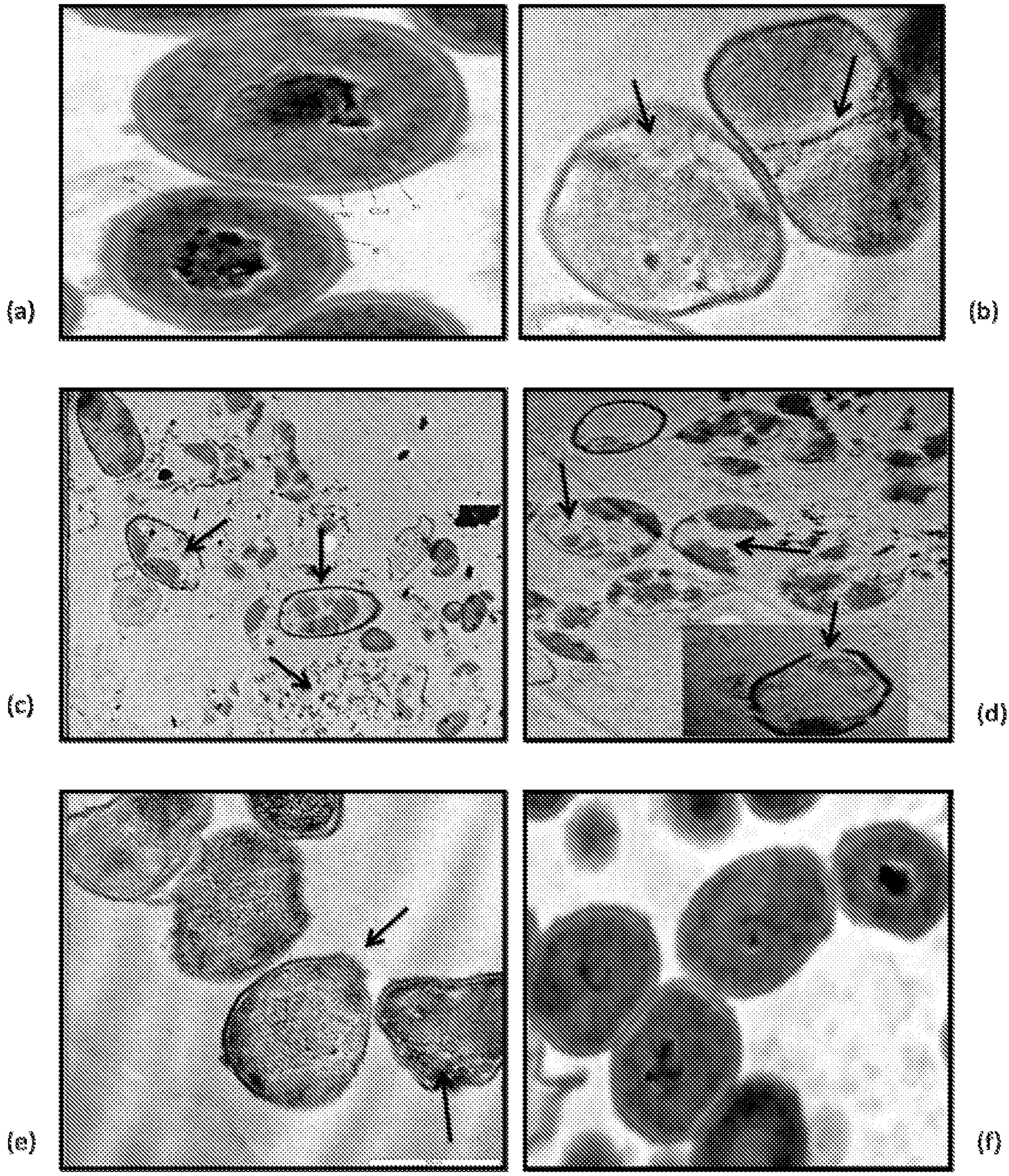


Figure 2

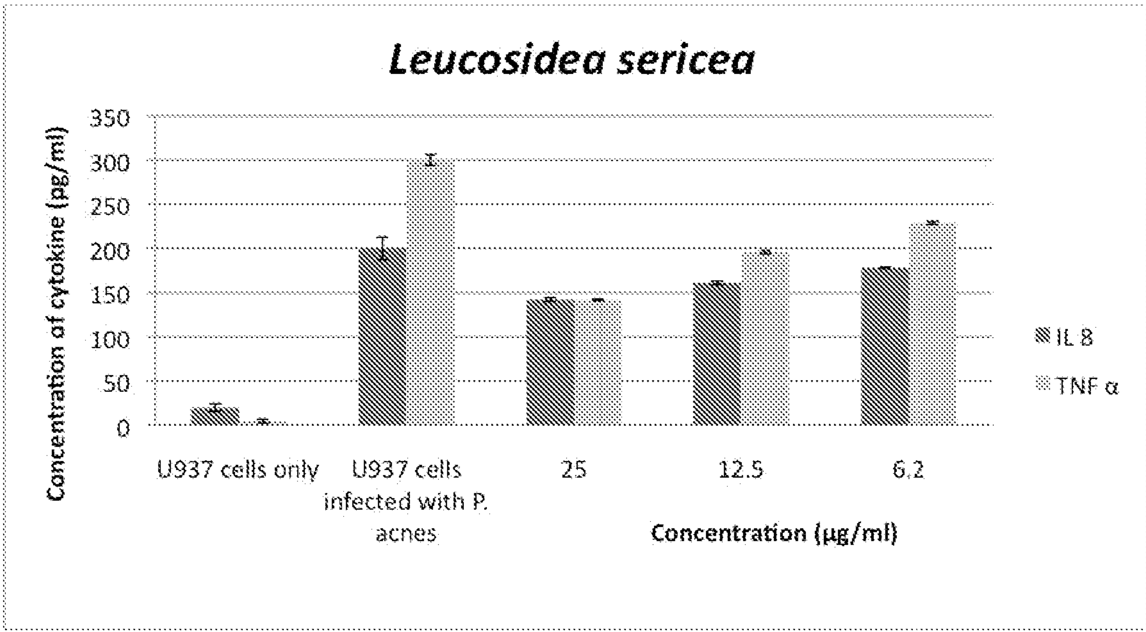
Leucosidea sericea
Concentration of cytokine (pg/ml)
350
300
250
200
150
100
50
0
U937 cells only
U937 cells infected with P. acnes
25
12.5
6.2
Concentration (µg/ml)
IL 8
TNF α

(a)

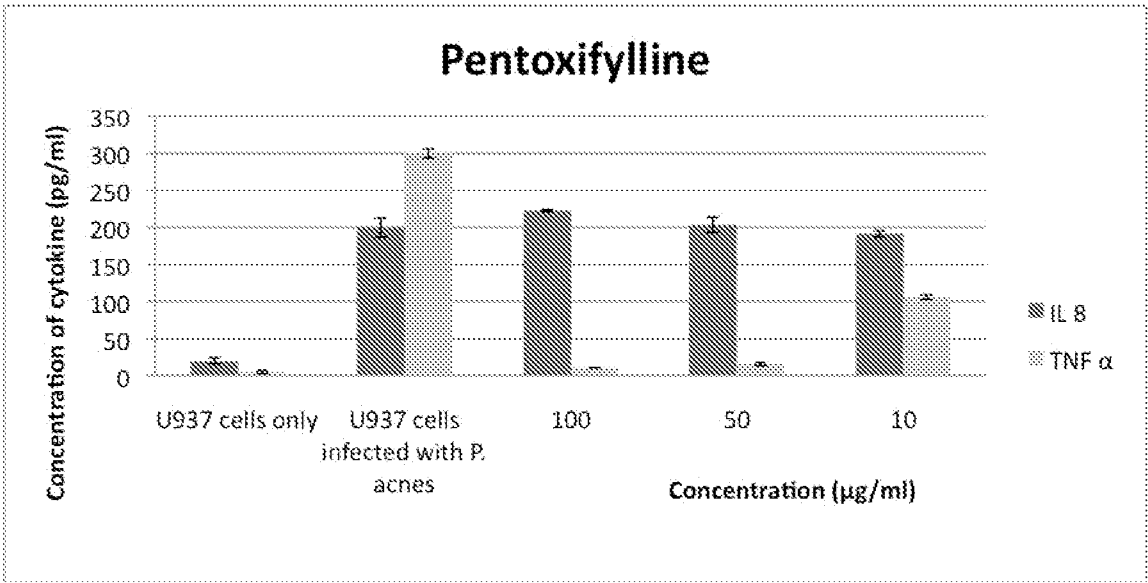
Pentoxifylline
Concentration of cytokine (pg/ml)
350
300
250
200
150
100
50
0
U937 cells only
U937 cells infected with P. acnes
100
50
10
Concentration (µg/ml)
IL 8
TNF α

(b)

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ARICAN, O. ; KURUTAS, E.B. ; SASMAZ S.** Oxidative stress in patients with acne vulgaris. *Mediators Inflamm,* 2005, vol. 6, 380-384
- **CHOMNAWANG, M.T ; SURASSMO, S. ; NUKOOLKARN, V.S. ; GRITSANAPAN, W.** Antimicrobial effects of Thai medicinal plants against acne-inducing bacteria. *J Ethnopharmacol,* 2005, vol. 101, 330-333
- **COENYE, T. ; BRACKMAN, G. ; RIGOLE, P. ; WITTE, E.D. ; HONRAET, K. ; ROSSEL, B. ; NELIS, H.J.** Eradication of Propionibacterium acnes biofilms by plant extracts and putative identification of icariin, resveratrol and salidroside as active compounds. *Phytomedicine,* 2012, vol. 19, 409-412
- **D'HELLENCOURT, C.L. ; DIAW, L. ; CORNILLET, P. ; GUENOUNOU, M.** Differential regulation of TNFa, IL-1$\beta$, IL-6, IL-8, TNF$\beta$ and IL-10 by pentoxifylline. *Int J Immunopharmac,* 1996, vol. 18, 739-748
- **DU TOIT, R. ; VOLSTEEDT, Y. ; APOSTOLIDES, Z.** Comparison of the antioxidant content of the fruits, vegetables and teas measured as Vitamin C equivalents. *Toxicology,* 2001, vol. 166, 63-69
- **ITOH, D. ; KAWANO, K. ; NABETA, K.** Biosynthesis of chloroplastidic and extrachloroplastidic terpenoids in liverwort cultured cells: C serine as a probe of terpene biosynthesis via mevalonate and non-mevalonate pathways. *J. Nat. Prod.,* 2003, vol. 66, 332-336
- **KUETE, V.** Potential of Cameroonian plants and derived-products against microbial infections. *A review. Planta Med,* 2010, vol. 76, 1479-1491
- **LEYDON, J.J.** Therapy for Acne vulgaris. *The New England J. Med,* 1997, 1156-1162
- **MAPUNYA, M.B. ; HUSSEIN, A.A. ; RODRIGUEZ, B. ; LALL, N.** Tyrosinase activity of Greyia flanaganii (bolus) constituents. *Phytomedicine,* 2011, vol. 18, 1006-1012
- **PAN, C.Y. ; CHEN, J.Y. ; LIN, T.L. ; LIN, C.H.** In vitro activities of three synthetic peptides derived from epinecidin-1 and an anti-lipopolysaccharide factor against Propionibacterium acnes, Candida albicans, and Trichomonas vaginalis. *Peptides,* 2009, vol. 30, 1058-1068
- **RIOS, M.Y. ; GONZALEZ-MORALES, A. ; VILLARREAL, L.** Sterols, triterpenes and biflavonoids of Viburnum jucundum and cytotoxic activity of ursolic acid. *Planta Med,* 2001, vol. 67, 683-684
- **SHAW, L. ; KENNEDY, C.** The treatment of acne. *J Paediatr Child Health,* 2007, vol. 17, 385-389
- **SULIMAN, S. ; VAN VUUREN S.F.** Validating the in vitro antimicrobial activity of Artemisia afra in polyherbal combinations to treat respiratory infections. *S. Afr. J. Bot.,* 2010, vol. 76, 655-661
- **SUNG, J.H. ; LEE, J.O. ; SON, J.K. ; PARK, N.S. ; KIM, M.R. ; KIM, J.G. ; MOON, D.C.** Cytotoxic constituents from Solidago virga-aurea var. gigantea MIQ. *Arch Pharm Res,* 1999, vol. 22 (6), 633-637
- **TANGHETTI, A.** Acne vulgaris and Rosacea: Optimizing Therapy. *Skin Disease Education Foundation's 29th Annual Hawaii Dermatology Seminar,* 18 March 2005
- **TSAI, T.H. ; TSAI, T.H. ; WU, W.H. ; TSENG, J.P. ; TSAI, P.J.** In vitro antimicrobial and anti-inflammatory effects of herbs against Propionibacterium acnes. *Food Chem,* 2010, vol. 119, 964-968
- **TSAI, I.L. ; CHENG, M.J. ; HUNG, H.W. ; CHENG, H.I. ; CHEN, I.S.** Chemical constituents from the leaves of Litsea acutivena. *J. Chinese Chem. Soc.,* 2007, vol. 54, 503-506
- **VAN WYK, B. ; VAN WYK, P. ; VAN WYK, B.E.** Photo Guide to Trees of Southern Africa. Briza Publications, 2008
- **LOUNASMAA, M. ; WIDEN, C.J. ; HUHTIKANGAS, A.** Phloroglucinol derivatives of Hagenia abyssinica. *Phytochem.,* 1973, vol. 12, 2017-2025
- **WOLDEMARIAM, T.Z. ; FELL, A.F. ; LINLEY, P.A. ; BIBBY, M.C. ; PHILLIPS, R.M.** Evaluation of the anti-tumour action and acute toxicity of kosins from Hagenia abyssinica. *J. of Pharmaceut. Biomed. Anal.,* 1992, vol. 10, 555-560